# EUROPEAN PATENT APPLICATION

(11) **EP 4 706 505 A1**
(43) Date of publication of application: **11.03.2026**
(21) Application number: 24807415.5
(22) Date of filing: 07.05.2024
(51) Int. Cl.: A61B 5/00, G16H 50/20

(54) **APPARATUS AND METHOD FOR DIAGNOSING BREAST CANCER**

(30) Priority: 15.05.2023 KR 20230062193
(71) Applicant: Olive Healthcare Inc., Seoul 05836 (KR)
(72) Inventor: HAN, Sung Ho, Seoul 05705 (KR); HONG, Hee Sun, Seoul 06763 (KR)
(74) Representative: Manitz Finsterwald Patent- und Rechtsanwaltspartnerschaft mbB
(86) International application number: PCT/KR2024/006081
(87) International publication number: WO 2024/237538

(57) **Abstract**

A breast cancer diagnosis apparatus according to the present invention comprises: a memory in which a breast cancer diagnosis program is stored; and a processor configured to execute the breast cancer diagnosis program,
wherein the breast cancer diagnosis program is configured to sequentially irradiate a subject with light of multiple wavelengths in the near-infrared region, receive output light from the subject for each wavelength according to the irradiation, and calculate a breast cancer index using the intensity of the output light for each wavelength,
wherein the breast cancer index is calculated based on a ratio of the output light from a reference region of the subject to the output light from a diagnostic target region.

## Description

### BACKGROUND

### Field

The present invention relates to a breast cancer diagnosis apparatus and method, and more particularly, to an apparatus and method for diagnosing breast cancer using near-infrared measurement technology.

Early diagnosis and treatment of diseases are important for maintaining a healthy life. Among various diseases, cancer is a serious disease that can threaten life, and interest in early diagnosis and treatment of cancer is increasing.

In women, among cancers such as breast cancer, thyroid cancer, gastric cancer, colorectal cancer, and lung cancer, the incidence rate of breast cancer is the highest. Accordingly, attention has been focused on seeking a method capable of effectively diagnosing breast cancer at an early stage in order to effectively treat breast cancer. As a conventional technique for diagnosing breast cancer, mammography using X-rays to detect lesions present inside the breast is known. However, in mammography, it is difficult to distinguish cancer from breast tissue because the difference in X-ray absorption rate between breast tissue and cancer is very small.

As a solution to such problems, there is a method of diagnosing breast cancer using diffuse optical tomography, which acquires two-dimensional or three-dimensional images of tissues inside the breast by using wavelengths of light.

For example, Korean Laid-open Patent Publication No. 10-2019-0048249 discloses an apparatus and method for diagnosing breast cancer using diffuse optical tomography.

Specifically, Korean Laid-open Patent Publication No. 10-2019-0048249 discloses an apparatus for diagnosing breast cancer, in which optical signals and signals obtained by detecting infrared light irradiated to a subject using the optical signals are respectively converted into digital signals, and a difference in signal magnitude and a phase difference of the converted digital signals are calculated.

### SUMMARY

An object of the present invention is to provide an apparatus and method for diagnosing breast cancer using DMW-NIRS (Discrete Multi Wavelength Near Infra-Red Spectroscopy), which employs light of multiple wavelengths in the near-infrared region.

The problems of the present invention are not limited to those mentioned above, and other problems not described herein will be clearly understood by those skilled in the art from the following description.

As a technical means for achieving the above-described problems, a breast cancer diagnosis apparatus according to an aspect of the present invention includes: a memory in which a breast cancer diagnosis program is stored; and a processor that executes the breast cancer diagnosis program. The breast cancer diagnosis program sequentially irradiates light of multiple wavelengths in the near-infrared region to a subject, receives output light from the subject according to the irradiation of the light for each wavelength, calculates a breast cancer index using the intensity of the output light for each wavelength, and calculates the breast cancer index based on a ratio of the output light from a reference region for a diagnostic target to the output light from a diagnostic target region.

A breast cancer diagnosis method using the breast cancer diagnosis apparatus according to an aspect of the present invention includes: sequentially irradiating light of multiple wavelengths in the near-infrared region to a subject; receiving output light from the subject according to the irradiation of the light for each wavelength; and calculating a breast cancer index using the intensity of the output light for each wavelength. The step of calculating the breast cancer index comprises calculating the breast cancer index based on a ratio of the output light from a reference region for a diagnostic target to the output light from a diagnostic target region.

According to the problem-solving means of the present invention described above, the breast cancer diagnosis apparatus according to the present invention provides the effect of easily identifying breast cancer by comparing the data of the output light collected through scanning of the diagnostic region with the data of the output light collected through scanning of the reference region.

### BRIEF DESCRIPTION OF THE DRAWINGS

FIG. 1 is a block diagram illustrating a breast cancer diagnosis apparatus according to an embodiment of the present invention.
FIG. 2 is a diagram illustrating a configuration of a control unit in the diagnostic apparatus according to an embodiment of the present invention.
FIGS. 3 to 5 are diagrams illustrating an example of a probe according to an embodiment of the present invention.
FIGS. 6 and 7 are diagrams illustrating main interfaces output on a display during breast cancer diagnosis according to an embodiment of the present invention.
FIG. 8 is a flowchart illustrating a process of calculating a breast cancer index according to an embodiment of the present invention.

### DETAILED DESCRIPTION

Hereinafter, embodiments of the present application will be described in detail with reference to the accompanying drawings so that those skilled in the art to which the present application pertains can easily carry out the invention. However, the present application may be implemented in various different forms and is not limited to the embodiments described herein. In the drawings, parts not related to the description are omitted for clarity, and similar reference numerals are assigned to similar parts throughout the specification.

Throughout this specification, when a certain part is described as being "connected" to another part, this includes not only the case of being "directly connected" but also the case of being "electrically connected" with another element interposed therebetween.

Throughout this specification, when a member is described as being "on" another member, this includes not only the case where the member is in contact with the other member but also the case where another member is present between the two members.

Hereinafter, preferred embodiments of the present invention will be described in detail with reference to the accompanying drawings and the content set forth below. However, the present invention is not limited to the embodiments described herein and may be embodied in other forms. Identical reference numerals throughout the specification denote identical components.

Hereinafter, the configuration of a breast cancer diagnosis apparatus according to an embodiment of the present invention will be described.

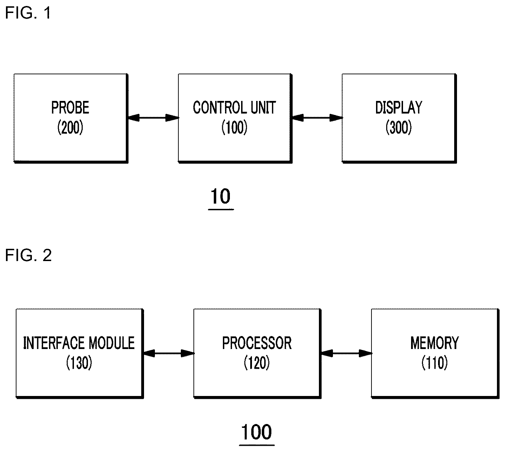

FIG. 1 is a block diagram illustrating a breast cancer diagnosis apparatus according to an embodiment of the present invention.

Referring to FIG. 1, the breast cancer diagnosis apparatus (10) of the present invention includes a probe (200) that emits light of multiple wavelengths in the near-infrared region to a subject and receives output light from the subject, and a control unit (100) that receives the optical data of the output light received by the probe (200) and calculates a breast cancer index of the subject based on the optical data. The breast cancer diagnosis apparatus (10) may further include a display (300) for outputting the breast cancer index calculated by the control unit (100).

FIG. 2 is a diagram illustrating the configuration of a control unit in the diagnostic apparatus according to an embodiment of the present invention.

The control unit (100) includes a memory (110) in which a breast cancer diagnosis program is stored, a processor (120) that executes the breast cancer diagnosis program, and an interface module (130).

The memory (110) stores the breast cancer diagnosis program. The breast cancer diagnosis program is configured to sequentially irradiate a subject with light of multiple wavelengths in the near-infrared region, receive output light from the subject in response to the irradiation for each wavelength, and calculate a breast cancer index using the intensity of the output light for each wavelength. At this time, the breast cancer diagnosis program calculates the breast cancer index based on the ratio of the output light from a diagnostic target region to the output light from a reference region for a diagnostic target, the details of which will be described later. In addition, the memory (110) functions to temporarily or permanently store data processed by the processor (120). Here, the memory (110) may include volatile storage media or non-volatile storage media; however, the scope of the present invention is not limited thereto.

The processor (120) executes the breast cancer diagnosis program stored in the memory (110). The processor (120) may refer to a data processing device embedded in hardware, having physically structured circuits to perform functions expressed as codes or instructions included in the program. Examples of such hardware-embedded data processing devices include microprocessors, central processing units (CPUs), processor cores, multiprocessors, application-specific integrated circuits (ASICs), and field programmable gate arrays (FPGAs). However, the scope of the present invention is not limited thereto.

The interface module (130) may communicate with external devices or various computing devices. The interface module (130) may be a device that includes hardware and software required to transmit and receive signals, such as control signals or data signals, through wired or wireless connections with external devices. For example, the interface module (130) may communicate with external devices via a local area network (LAN), a wide area network (WAN), the Internet (WWW), or wired/wireless data communication networks.

It should be noted that the components illustrated in FIG. 2 according to the embodiment of the present invention represent hardware components such as software, FPGA (Field Programmable Gate Array), or ASIC (Application-Specific Integrated Circuit), and perform predetermined roles. However, the term "components" is not limited to software or hardware, and each component may be configured to reside in addressable storage media or to reproduce one or more processors. For example, the components may include software components, object-oriented software components, class components, and task components, as well as processes, functions, attributes, procedures, subroutines, program code segments, drivers, firmware, microcode, circuits, data, databases, data structures, tables, arrays, and variables. The components and the functions provided therein may be combined into a smaller number of components or further divided into additional components.

Now, the detailed configuration of the probe (200) will be described.

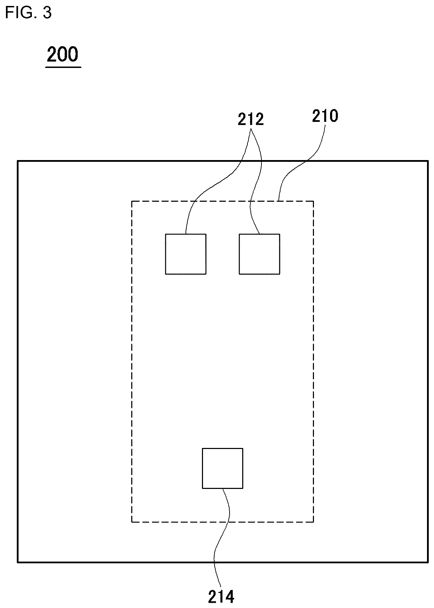

FIGS. 3 to 5 are diagrams illustrating an example of a probe according to an embodiment of the present invention.

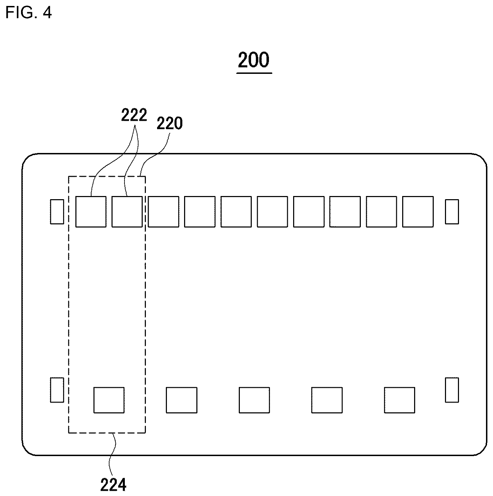

The probe (200) sequentially irradiates a subject (20) with light of multiple wavelengths in the near-infrared region and receives output light from the subject (20) in response to the irradiation. To this end, the probe (200) includes a light emitting element (212) and a light receiving element (214). At this time, the light emitting element (212) and the light receiving element (214) form a pair to constitute a channel signal processor (210). As shown in FIG. 4, a plurality of channel signal processors (220) may be arranged on one surface of the probe.

Meanwhile, the probe (200) is grasped by a user of the breast cancer diagnosis apparatus (10), and for easy gripping, it may have various small-sized shapes. In addition, it may be connected to the control unit (100) by wired or wireless communication, receive control signals from the control unit (100), and transmit data on the output light of each wavelength to the control unit (100).

The probe (200) includes at least one channel signal processor (210, 220). In particular, as shown in FIG. 4, a plurality of channel signal processors (220) that receive output light from different regions of the subject (20) may be adjacently arranged so that optical data from a predetermined area of the breast of the subject (20) can be obtained through a single measurement.

The channel signal processors (210, 220) may be arranged in the probe (200) in a number N (N is a natural number of 1 or more) and, as described above, include pairs of light emitting elements (212) and light receiving elements (214). The channel signal processors process the measurement values (hereinafter referred to as optical data) of the output light from the subject (20) detected through the light receiving elements (214, 224). Here, the near-infrared light incident on the subject (20) may be absorbed or scattered by biological tissue, and by analyzing the output light from the subject (20), the absorption or scattering state in the biological tissue can be analyzed.

The light emitting elements (212, 222) may include optical output elements such as LDs (laser diodes), LEDs (light emitting diodes), or VCSELs (vertical cavity surface emitting lasers) that can output incident light having different wavelengths within the near-infrared region. In the present invention, it is preferable that the light emitting elements (212, 222) be configured to output at least eight different wavelengths of light. Accordingly, if the light emitting elements (212, 222) can output eight different wavelengths of light, one light emitting element (212, 222) may be included in each channel signal processor (210, 220). If the light emitting elements (212, 222) can output four different wavelengths of light, the light emitting elements (212, 222) outputting the first to fourth wavelengths and the light emitting elements (212, 222) outputting the fifth to eighth wavelengths may be arranged in the channel signal processors (210, 220).

In the case of FIG. 4, N channel signal processors (220) are arranged in a horizontal direction along one axis of the probe (200), and the light emitting elements (222) and light receiving elements (224) of adjacent channel signal processors (220) are arranged close to each other, such that the light emitting elements (222) are aligned in a row parallel to the axis, and the light receiving elements (224) are arranged in a row parallel to the axis at a predetermined distance from the light emitting elements (222). At this time, the penetration depth of near-infrared light can be adjusted by the distance between the light receiving element (224) and the light emitting element (222), and accordingly, the measurement depth in the subject can be adjusted. For example, the penetration depth of near-infrared light is about half of the separation distance. Therefore, by setting an appropriate separation distance between the light receiving element (224) and the light emitting element (222), an optimal measurement depth can be set. In other words, by configuring the distance between the light emitting element (222) and the light receiving element (224) included in each channel signal processor (220) to be the same, the plurality of light emitting elements (222) may be arranged in a row in a first direction on the contact surface of the probe (200), and the plurality of light receiving elements (224) may be arranged in a row in a second direction parallel to the first direction on the contact surface of the probe (200). As such, by including one or more channel signal processors (220), the probe (200) can collect optical data over a wide area of the subject (20) with a single contact when collecting optical data from the subject (20).

Each channel signal processor (210, 220) may include a driving chip (not shown) that sequentially outputs driving signals for driving the light emitting elements (212, 222).

The number of types of incident light by wavelength output by the light emitting elements (212, 222) may be determined based on the number of types of chromophores present inside the breast. Since chromophores effectively absorb light having specific wavelengths depending on their type, by irradiating the breast with incident light having the wavelengths effectively absorbed by each type of chromophore, and collecting and analyzing the output light, the concentration of each type of chromophore present in the breast can be measured.

The light receiving elements (214, 224) may be optical collection devices such as photodiodes, photo transistors, photomultiplier tubes (PMTs), or photocells that can receive light and collect optical data. The optical data of the output light collected by the light receiving elements (214, 224) is transmitted to the control unit (100) and used to calculate the breast cancer index.

The light emitting elements (212, 222) and the light receiving elements (214, 224) are installed in the probe (200) to be exposed on a predetermined surface of the probe (200), so that when the contact surface of the probe (200) comes into contact with the subject (20) such as a breast, the light emitting elements (212, 222) and the light receiving elements (214, 224) come into contact with the subject (20). Then, the incident light irradiated from the light emitting elements (212, 222) passes through the subject (20), or is reflected, absorbed, or scattered in the subject (20), and the output light of the subject reflecting these processes is detected by the light receiving elements (214, 224). The light emitting elements (212, 222) emit near-infrared light at different times for each wavelength, and accordingly, the light receiving elements (214, 224) collect optical data of different wavelengths from the subject, which may include information on the wavelength and intensity of the output light.

In addition, the outer housing of the probe (200) may further include a notification unit such as a blinking LED or a liquid crystal display. The notification unit performs a function of informing the user of the operating state of the probe (200). For example, when the probe (200) completes collection of optical data from the subject (20), it may notify the user of the completion of optical data collection of the probe (200) by blinking the LED or displaying information about the completion of optical data collection on the liquid crystal display.

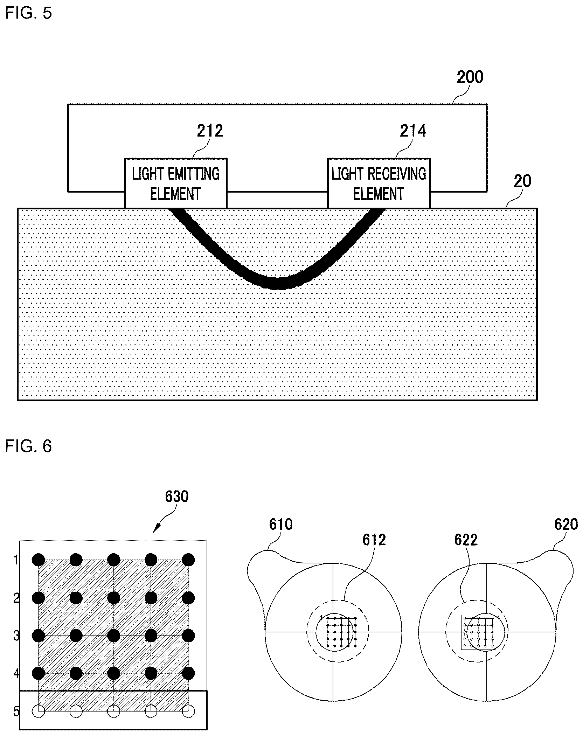

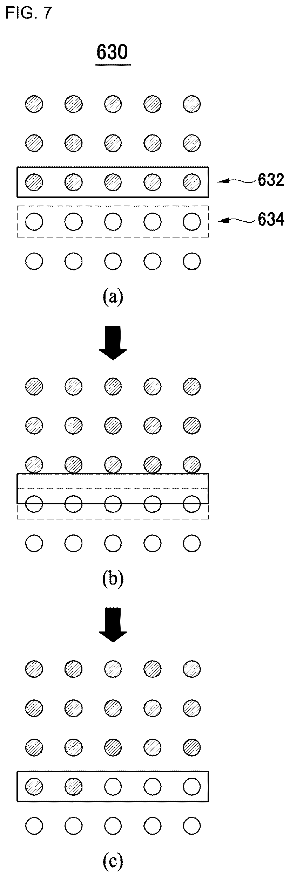

FIGS. 6 and 7 are diagrams illustrating main interfaces output on a display during breast cancer diagnosis according to an embodiment of the present invention.

The display (300), under the control of the control unit (100), outputs a region setting interface (610, 620) and a scan guide interface (630) for breast cancer diagnosis. The region setting interfaces (610, 620) respectively display the left breast region and the right breast region, and are used to set a scan region (612) serving as the diagnostic target and a scan region (622) serving as the reference region.

Prior to performing breast cancer diagnosis according to the present invention, an examiner may determine a suspicious region requiring further diagnosis by palpation or ultrasound examination, and this region is set as the scan region (612) serving as the diagnostic target in the present invention. A region in the contralateral breast of the same subject at the same or similar position as the scan region may be set as the scan region (622) serving as the reference region. That is, by comparing the output light data collected from the suspicious region and from a normal region, a breast cancer index is calculated and breast cancer in the suspicious region is diagnosed. Accordingly, in the region setting interfaces (610, 620), the basic positions of the left and right breasts are displayed, and a first ROI interface (612) representing the diagnostic target region in a first coordinate system and a second ROI interface (622) representing the reference region in a second coordinate system are displayed. At this time, the reference region may be set to a region symmetrical to the diagnostic target region.

The first ROI interface (612) may be directly set by a user of the breast cancer diagnosis apparatus (10). Meanwhile, the second ROI interface (622) is provided for scanning the reference region for comparison, and may be directly set by the user or automatically set to the mirror-reflected region corresponding to the first ROI interface (612) after it is set.

The scan guide interface (630) displays the process of collecting output light data through the probe (200) described above. The scan guide interface (630) guides the position or scanning direction of the probe (200) so that the user can collect optical data of the breast using the probe (200). In addition, each coordinate system of the first ROI interface (612) or the second ROI interface (622) may be enlarged and displayed as a third coordinate system.

Specifically, when the user collects optical data corresponding to the first ROI interface (612) or the second ROI interface (622) using the probe (200), the scan guide interface (630) displays the optical data collected by each of the plurality of channel signal processors (220) in a two-dimensional coordinate form.

FIG. 7 illustrates this scanning process in more detail.

As shown in FIG. 4, when the probe (200) includes five channel signal processors (220), the optical data collected by each channel signal processor (220) is displayed as coordinates (circles) in the scan guide interface (630). In particular, a circle displayed in black or gray indicates that optical data at that point has been collected, while a circle displayed in white indicates that optical data at that point has not yet been collected.

As shown in FIG. 7(a), when collection of optical data for a particular row is completed, the scan guide interface (630) indicates that the probe (200) should be scanned downward to collect optical data for the row below. For example, each circle of the completed row may be temporarily lit or flashed to indicate completion of optical data collection for that row. The solid box (632) indicating the current position of the probe (200) and the dotted box (634) indicating the position to be scanned by the probe (200) are displayed to guide the movement of the probe (200). That is, as shown in FIG. 7(a), when scanning at the current position of the probe (200) is completed, the dotted box (634) is moved and displayed to the row below. Then, as shown in FIG. 7(b), the user is guided to move the solid box (632) to the dotted box (634) using the probe (200), and as shown in FIG. 7(c), optical data is collected by scanning at the changed position.

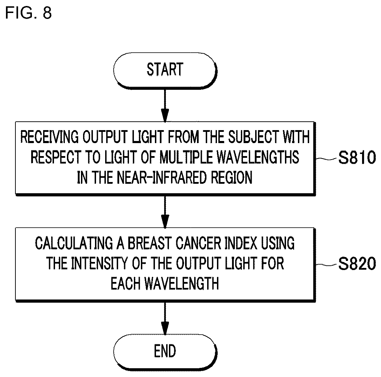

FIG. 8 is a flowchart illustrating a process of calculating a breast cancer index according to an embodiment of the present invention.

First, the control unit (100) sequentially irradiates the subject with multiple wavelengths of light in the near-infrared region, and receives the output light from the subject for each wavelength according to the irradiation (S810). As described above, a scan region for the diagnostic target and a scan region used as a reference region are specified, and scanning is performed on these regions to receive the output light.

Next, the breast cancer index is calculated based on the ratio of the output light from the diagnostic target region to the output light from the reference region for a diagnostic target (S820).

The control unit (100) calculates the ratio of the output light from the diagnostic target region to the output light from the reference region for the diagnostic target for each of the multiple wavelengths, and may calculate the breast cancer index by applying various arithmetic operations (e.g., the four basic operations) to each wavelength ratio.

Meanwhile, the breast cancer index may be calculated for each measurement coordinate, as shown in FIG. 6 or FIGS. 7(a) to 7(c), and the average value or maximum value of the breast cancer indices at each measurement coordinate in the region of interest may be compared with a threshold value to finally diagnose the presence of breast cancer.

In addition, unlike the above embodiment, another embodiment may be considered in which output light for each measurement coordinate is used for the diagnostic target region, while the average value of output light for each measurement coordinate is used for the reference region. When comparing output light for each coordinate individually, there is a risk of large deviations, so the average value of the output light at each coordinate is used for the reference region. Accordingly, output light for each coordinate and each wavelength is used for the diagnostic target region, while the average value of output light for each wavelength obtained from all coordinates is used for the reference region.

Conventionally, methods have been known that calculate the concentration of chromophores contained in the subject based on light data output from the subject according to irradiation of light. That is, the absorption coefficient and scattering coefficient of a turbid medium in the near-infrared region are measured, and the concentration of chromophores contained in the turbid medium is calculated. For example, the concentrations of four chromophores-water (H2O), lipid, oxyhemoglobin (O2Hb), and deoxyhemoglobin (HHb)-are calculated, and biological information is analyzed based on their distribution.

In the present invention, instead of using a method of calculating the concentration of individual chromophores, the breast cancer index can be calculated simply by using the ratio of the output light from the diagnostic target region to the output light from the reference region. Comparing chromophore values of tumors and normal tissues shows that in tumor tissues, hemoglobin (both oxyhemoglobin and deoxyhemoglobin) increases due to angiogenesis compared to normal tissues, and spectral changes suggesting not only an increase in water but also changes in water binding states have been observed. It is also known that in tumors, the values of lipids and oxygen saturation decrease compared to normal tissues. The present invention uses these characteristics to calculate the breast cancer index based on the output light from the lesion and normal regions, representing the ratio of chromophores between the two regions.

Thus, the breast cancer diagnosis apparatus according to the present invention provides the effect of conveniently scanning the breast, since the probe is equipped with multiple channel signal processors and can collect optical data from a wide area of the breast at once.

In addition, the invention improves convenience of the scanning process by providing a region setting interface and a scan guide interface that allow precise scanning of the breast with the probe.

Furthermore, by comparing the output light data collected through scanning of the diagnostic target region with the output light data collected through scanning of the reference region, the invention provides the effect of easily identifying breast cancer.

The breast cancer diagnosis method according to an embodiment of the present invention may also be implemented in the form of a computer-readable medium containing computer-executable instructions, such as program modules, executed by a computer. The computer-readable medium may be any available medium accessible by a computer, including volatile and non-volatile media, removable and non-removable media. In addition, the computer-readable medium may include computer storage media. The computer storage media include volatile and non-volatile, removable and non-removable media implemented in any method or technology for storage of information such as computer-readable instructions, data structures, program modules, or other data. Although the method and system of the present invention have been described with reference to specific embodiments, all or part of their components or operations may be implemented using computer systems with general-purpose hardware architectures.

The foregoing description of the present invention is for illustrative purposes, and those skilled in the art will understand that various changes in form and details can be made without departing from the spirit or essential characteristics of the invention. Therefore, the embodiments described above should be understood as illustrative and not restrictive in all respects. For example, each component described as a single unit may be implemented in a distributed manner, and likewise, components described as distributed may be implemented in a combined form.

The scope of the present invention should be defined not by the detailed description above, but by the appended claims, and all modifications or variations derived from the meaning and scope of the claims and their equivalents should be construed as being included within the scope of the present invention.

## Claims

1. A breast cancer diagnosis apparatus, comprising:
a memory in which a breast cancer diagnosis program is stored; and
a processor configured to execute the breast cancer diagnosis program,
wherein the breast cancer diagnosis program is configured to sequentially irradiate a subject with light of multiple wavelengths in the near-infrared region, receive output light from the subject for each wavelength according to the irradiation, and calculate a breast cancer index using the intensity of the output light for each wavelength,
wherein the breast cancer index is calculated based on a ratio of the output light from a reference region for a diagnostic target to the output light from a diagnostic target region.

2. The breast cancer diagnosis apparatus of claim 1,
wherein the breast cancer diagnosis program is configured to calculate the breast cancer index based on values obtained by calculating ratios of the output light from the reference region to the output light from the diagnostic target region for each of multiple wavelengths.

3. The breast cancer diagnosis apparatus of claims 1 or 2,
wherein the breast cancer index is calculated by measuring, for each coordinate of a region of interest, the output light from the reference region and the output light from the diagnostic target region.

4. The breast cancer diagnosis apparatus of claims 1 or 2,
wherein the breast cancer index is calculated by measuring, for each coordinate of a region of interest, the output light from the reference region and the output light from the diagnostic target region,
and calculating the breast cancer index based on a ratio of an average value of the output light for each coordinate of the reference region to the output light for each coordinate of the diagnostic target region in the region of interest.

5. The breast cancer diagnosis apparatus of claim 1,
further comprising a probe configured to sequentially output light of multiple wavelengths in the near-infrared region to the subject, receive output light from the subject, and sequentially process the output light.

6. The breast cancer diagnosis apparatus of claim 1,
wherein the breast cancer diagnosis program is configured to output a region setting interface for setting a diagnostic region of breast cancer,
wherein the region setting interface includes a first ROI interface indicating a scan region serving as a diagnostic target and a second ROI interface indicating a scan region serving as a reference region, through a region setting interface that displays a left breast region and a right breast region, respectively.

7. A breast cancer diagnosis method using a breast cancer diagnosis apparatus, comprising:
irradiating a subject sequentially with light of multiple wavelengths in the near-infrared region and receiving output light from the subject for each wavelength according to the irradiation; and
calculating a breast cancer index using the intensity of the output light for each wavelength,
wherein calculating the breast cancer index comprises calculating the breast cancer index based on a ratio of the output light from a reference region for a diagnostic target to the output light from a diagnostic target region.

8. The breast cancer diagnosis method of claim 7,
wherein calculating the breast cancer index comprises calculating the breast cancer index based on values obtained by calculating ratios of the output light from the reference region to the output light from the diagnostic target region for each of multiple wavelengths.

9. The breast cancer diagnosis method of claims 7 or 8,
wherein the breast cancer index is calculated by measuring, for each coordinate of a region of interest, the output light from the reference region and the output light from the diagnostic target region.

10. The breast cancer diagnosis method of claims 7 or 8,
wherein calculating the breast cancer index comprises measuring, for each coordinate of a region of interest, the output light from the reference region and the output light from the diagnostic target region,
and calculating the breast cancer index based on a ratio of an average value of the output light for each coordinate of the reference region to the output light for each coordinate of the diagnostic target region in the region of interest.

11. The breast cancer diagnosis method of claim 7,
wherein receiving the output light for each wavelength comprises outputting a region setting interface for setting a diagnostic region of breast cancer,
wherein the region setting interface includes a first ROI interface indicating a scan region serving as a diagnostic target and a second ROI interface indicating a scan region serving as a reference region, through a region setting interface that displays a left breast region and a right breast region, respectively.
